# EUROPEAN PATENT APPLICATION

(11) **EP 1 754 482 A1**
(43) Date of publication of application: **21.02.2007**
(21) Application number: 06123838.2
(22) Date of filing: 18.11.2002
(51) Int. Cl.: A61K 31/435, A61P 17/00

(54) **Use of an ascomycin for the treatment of blepharitis**

(30) Priority: 19.11.2001 EP 01127546
(62) Divisional of application: 02790400.2
(71) Applicant: Novartis AG, 4002 Basel (CH); Novartis Pharma GmbH, 1235 Vienna (AT)
(72) Inventor: Fsadni, Mario, Harpenden, Hampshire AL5 5UB (GB); Latour, Elisabeth, 75014, Paris (FR); Szczesny, Piotr, 8180, Bülach (CH)
(74) Representative: von Sprecher, Georg

(57) **Abstract**

This invention relates to the use of an ascomycin for the treatment of blepharitis.

## Description

This invention relates to the use of an ascomycin in the preparation of a medicament for the treatment of blepharitis.

Blepharitis is an inflammation of the margin of the eyelid, and is typically classified as staphylococcal blepharitis, seborrhoeic blepharitis or allergic blepharitis. Often patients present with a symptom typical of both staphylococcal and seborrhoeic blepharitis.

We have found that an ascomycin is surprisingly useful to treat eyelid complications, in particular blepharitis. More preferably, the use of the present invention refers to seborrhoeic blepharitis and allergic blepharitis and in particular allergic blepharitis.

As used herein an ascomycin refers to ascomycin itself or a derivative, antagonist, agonist or analogue thereof, e.g. a compound of the FK 506 class.

Preferred ascomycins for use in the present invention include FK506 or a derivative, antagonist, agonist or analogue of FK506, which retain the basic structure and modulate at least one of the biological properties (for example immunological properties) of FK506 such as described in e.g. EP 184162, EP 315978, EP 323042, EP 423714, EP 427680, EP 465426, EP 474126, WO 91/13889, WO 91/19495, EP 484936, EP 532088, EP 532089, EP 569337, EP 626385, WO 93/5059 and the like; 33-epi-chloro-33-desoxy-ascomycin as disclosed in Example 66a in EP 427680 (hereinafter referred to as Compound A); {[1E-(1R,3R,4R)]1R,4S,5R,6S,9R,10E,13S,15S,16R,17S,19S,20S}-9-ethyl-6,16, 20-trihydroxy-4-[2-(4-hydroxy-3-methoxy-cyclohexyl)-1-methylvinyl]-15,17-dimethoxy-5,11,13,19-tetramethyl-3-oxa-22-aza-tricyclo[18.6.1.0(1,22)]heptacos-10-ene-2,8,21,27-tetraone as disclosed in Examples 6d and 71 in EP 569 337 (hereinafter referred to as Compound B); and {1R,5Z,9S,12S-[1E-(1R,3R,4R)],13R,14S,17R,18E, 21S,23S,24R,25S,27R}17-ethyl-1,14-dihydroxy-12-[2-(4-hydroxy-3-methoxy-cyclohexyl)-1-methylvinyl]-23,25-dimethoxy-13,19,21,27-tetramethyl-11,28-dioxa-4-aza-tricyclo[22.3.1.0(4,9)] octacosa-5,18-diene-2,3,10,16-tetraone, also known as 5,6-dehydro-ascomycin as disclosed in Example 8 in EP 626 385 (hereinafter referred to as Compound C); imidazolylmethyloxyascomycin, as disclosed in Example 1 and as compound of formula I in WO 97/08182 (hereinafter referred to as Compound D); 32-O-(1-hydroxyethylindol-5-yl)ascomycin, also known as Indolyl-ASC or L-732 531 as disclosed in Transplantation 65 (1998) 10-18, 18-26, on page 11, Figure 1 (hereinafter referred to as Compound E); or (32-deoxy-32-epi-N1-tetrazolyl)ascomycin, also known as ABT-281 as disclosed in J. Inv. Derm. 112 (1999), 729-738, on page 730, Figure 1 (hereinafter referred to as Compound F).

FK 506, Compounds A, B, C, D, E, and F are preferred ascomycins, more preferred are Compounds A, B, and C, especially FK506 and Compound A. Particularly preferred is Compound A.

In one aspect, the present invention is related to a method of treating blepharitis, which method comprises, for example the administration of a pharmaceutical composition comprising a therapeutically effective amount of an ascomycin to the affected area of the patient suffering from blepharitis, said ascomycin being preferably administered to the eyelid of said patient, said ascomycin being preferably but not exclusively formulated as an ointment, cream, gel, or lotion, which is preferably compatible with the ocular tissue.

During the administration of the pharmaceutical composition comprising an ascomycin to the affected area or to the eyelid of a patient suffering from blepharitis, it may occur that a portion of said composition comprising said ascomycin may come into contact with the ocular tissue of the eye, such as cornea, conjunctiva and the like. Therefore, ocular compatibility of the pharmaceutical composition used to treat blepharitis is a preferred aspect.

The present invention also pertains to the use of an ascomycin in the preparation of a pharmaceutical composition for the treatment of blepharitis.

Within the terms of this invention, all preferred embodiments and aspects as described hereinabove and hereinafter shall apply mutatis mutandis to a use and/or a method as disclosed hereinabove and hereinafter.

As used herein, the terms "treatment", "treat" or "treating" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition.

The mode of administration is critical for the ingredients and auxiliaries being present in a pharmaceutical composition comprising an ascomycin. The various modes of administering an ascomycin to a patient suffering from blepharitis are described infra. A highly preferred mode of administration is topical administration.

Pharmaceutical compositions for enteral, such as oral, rectal, parenteral, local, also topical administration typically comprise the pharmacological active ingredient on its own or together with other pharmaceutical excipients as required. The pharmaceutical compositions comprise, for example, approximately from 0.000'0001 % to 10%, preferably from approximately 0.0001% to approximately 1%, more preferably from approximately 0.001% to approximately 1% of the active ingredient.

Pharmaceutical compositions for enteral or parenteral and also for local administration are, for example, compositions in unit dose forms, such as dragées, tablets, capsules or suppositories, and also ampoules. Those compositions are prepared in a manner known per se, for example by means of conventional mixing, granulating, confectioning, dissolving or lyophilizing processes. For example, pharmaceutical compositions for oral administration can be obtained by combining the active ingredient with solid carriers, optionally granulating a resulting mixture and, if desired, processing the mixture or granules, if necessary after the addition of suitable excipients, to form tablets or dragée cores.

Suitable carriers for systemic administration are especially fillers, such as sugars, for example lactose, saccharose, mannitol or sorbitol, cellulose preparations and/or calcium phosphates, for example tricalcium phosphate or calcium hydrogen phosphate, also binders, such as starch pastes using, for example, corn, wheat, rice or potato starch, gelatin, gum tragacanth, methylcellulose and/or polyvinylpyrrolidone, and, if desired, disintegrators, such as the above-mentioned starches, also carboxymethyl starch, cross-linked polyvinylpyrrolidone, agar or alginic acid or a salt thereof, such as sodium alginate. Excipients are especially flow conditioners and lubricants, for example silicic acid, talc, stearic acid or salts thereof, such as magnesium or calcium stearate, and/or polyethylene glycol. Dragée cores are provided with suitable, optionally enteric, coatings, there being used, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the production of enteric coatings, solutions of suitable cellulose preparations, such as acetylcellulose phthalate or hydroxypropylmethylcellulose phthalate. Colourings or pigments may be added to the tablets or dragée coatings, for example for identification purposes or to indicate different doses of active ingredient.

Further orally administrable pharmaceutical compositions include dry-filled capsules consisting of gelatin, and also soft, sealed capsules consisting of gelatin and a plasticiser, such as glycerol or sorbitol. The dry-filled capsules may contain the active ingredient in the form of granules, for example in admixture with fillers, such as lactose, binders, such as starches, and/or glidants, such as talc or magnesium stearate, and optionally stabilisers. In soft capsules, the active ingredient is preferably dissolved or suspended in suitable liquids, such as fatty oils, paraffin oil or liquid polyethylene glycols, to which stabilisers may likewise be added.

Suitable rectally administrable pharmaceutical compositions are, for example, suppositories that consist of a combination of the active ingredient and a suppository base. Suitable suppository bases are, for example, natural or synthetic triglycerides, paraffin hydrocarbons, polyethylene glycols and higher alkanols. It is also possible to use gelatin rectal capsules that comprise a combination of the active ingredient and a base material. Suitable base materials are, for example, liquid triglycerides, polyethylene glycols and paraffin hydrocarbons.

For parenteral administration there are suitable especially aqueous solutions of an active ingredient in water-soluble form, for example in the form of a water-soluble salt, and also suspensions of the active ingredient, such as corresponding oily injection suspensions, there being used suitable lipophilic solvents or vehicles, such as fatty oils, for example sesame oil, or synthetic fatty acid esters, for example ethyl oleate or triglycerides, or aqueous injection suspensions that comprise viscosity-increasing substances, for example sodium carboxymethylcellulose, sorbitol and/or dextran, and, if desired, also stabilisers.

For topical administration, typically corresponding compositions are in general administered topically to the skin, preferably to the skin around the eye lid, and in particular to the eye lid, preferably to the skin of the eyelid, especially in the form of an ointment, cream, gel, or lotion. A preferred pharmaceutical composition used in the present invention is an ointment. The application of a skin cream comprising an ascomycin directly on to the skin of the eyelid is equally suitable.

Such compositions comprise the active ingredient (an ascomycin), for example, in a range of from approximately 0.000'0001 to approximately 10% by weight, preferably from approximately 0.0'000'1 to approximately 5% by weight, in particular from approximately 0.000'1 to approximately 1% by weight. Another preferred range is from 0.0001 to 0.05% by weight.

Generally, the dose of the active ingredient may depend on various factors, such as mode of administration, requirement, age and/or individual condition.

Topical compositions may further contain customary pharmaceutically acceptable excipients or additives known to the person skilled in the art, for example isotonising agents also called tonicity enhancers, buffers, complexing agents, solubilizers and thickeners.

Carriers for topical administration are for example water, mixtures of water and water-miscible solvents, such as C₁- to C₇-alkanols, vegetable oils or mineral oils, ethyl oleate, carboxymethyl-cellulose, polyvinyl-pyrrolidone and other non-toxic water-soluble polymers, in particular compatible with ocular tissue, such as, for example, cellulose derivatives, such as methylcellulose, alkali metal salts of carboxy-methylcellulose, hydroxymethylcellulose, hydroxyethylcellulose, methylhydroxypropyl-cellulose, hydroxypropylcellulose, chitosan and scleroglucan, acrylates or methacrylates, such as salts of polyacrylic acid or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as poloxamers, e.g. Poloxamer F127, polyvinyl alcohol, polyvinylpyrrolidone, polyvinyl methyl ether, polyethylene oxide, preferably cross-linked polyacrylic acid, such as neutral Carbopol, or mixtures of those polymers. The concentration of the carrier is, for example, from 0.1 to 100000 times the concentration of the active ingredient.

Solubilizers may also be used in a pharmaceutical composition for topical administration and are, for example, a cyclodextrin, tyloxapol, fatty acid glycerol polyethylene glycol esters, fatty acid polyethylene glycol esters, polyethylene glycols, glycerol ethers, polysorbate 20, polysorbate 80 or mixtures of those compounds. A specific example of an especially preferred solubilizer is a reaction product of castor oil and ethylene oxide, for example the commercial products Cremophor EL^{®}or Cremophor RH 40^{®}. Reaction products of castor oil and ethylene oxide have proved to be particularly good solubilizers that are compatible with ocular tissue. Other preferred solubilizers are cyclodextrins and tyloxapol. The concentration used depends especially on the concentration of the active ingredient. The amount added is typically sufficient to solubilize the active ingredient. For example, the concentration of the solubilizer is from 0.1 to 5000 times the concentration of the active ingredient.

Examples of buffers are acetate, ascorbate, borate, hydrogen carbonate / carbonate, citrate, gluconate, lactate, phosphate, propionate and TRIS (tromethamine) buffers. Tromethamine and borate buffer are preferred buffers. The amount of a buffer added is, for example, that necessary to ensure and maintain a physiologically tolerable pH range. The pH range is typically in the range of from 4 to 10, preferably of from 5 to 9, and also preferably from 6 to 8.5.

Tonicity enhancing agents are, for example, ionic compounds, such as alkali metal or alkaline earth metal halides, such as, for example, CaCl₂, KBr, KCI, LiCl, Nal, NaBr or NaCl, Na₂SO₄ or boric acid. Non-ionic tonicity enhancing agents are, for example, urea, glycerol, sorbitol, mannitol, propylene glycol, or dextrose. For example, sufficient tonicity enhancing agent is added to impart to the pharmaceutical composition a better compatibility with the ocular tissue, e.g. to impart an osmolality of approximately from 50 to 1000 mOsmol,

Examples of preservatives are quaternary ammonium salts such as e.g. sepazonium chloride, cetyltrimethylammonium bromide (cetrimide), cetylpyridinium chloride, benzoxonium chloride, benzethonium chloride, domiphen bromide (Bradosol^{®}) or benzalkonium chloride, alkyl-mercury salts of thiosalicylic acid, such as, for example, thiomersal, phenylmercuric nitrate, phenylmercuric acetate or phenylmercuric borate, parabens, such as, for example, methylparaben or propylparaben, alcohols, such as, for example, chlorobutanol, benzyl alcohol or phenyl ethanol, guanidine derivatives, such as, for example, chlorohexidine or polyhexamethylene biguanide, sodium perborate, Germal^{®}II or sorbic acid. Preferred preservatives are quaternary ammonium salts, alcohols, alkyl-mercury salts and parabens. Where appropriate, a sufficient amount of preservative is added to the pharmaceutical composition to ensure protection against secondary contaminations during use caused by bacteria and fungi.

The topical compositions may comprise further non-toxic excipients, such as, for example, emulsifiers, wetting agents or fillers, such as, for example, the polyethylene glycols designated 200, 300, 400 and 600, or Carbowax designated 1000, 1500, 4000, 6000 and 10000. Other excipients that may be used if desired are listed below but they are not intended to limit in any way the scope of the possible excipients. They are especially complexing agents, such as disodium-EDTA or EDTA, antioxidants, such as ascorbic acid, acetylcysteine, cysteine, sodium hydrogen sulfite, butyl-hydroxyanisole, butyl-hydroxytoluene or alpha-tocopherol acetate; stabilizers, such thiourea, thiosorbitol, sodium dioctyl sulfosuccinate or monothioglycerol; or other excipients, such as, for example, lauric acid sorbitol ester, triethanol amine oleate or palmitic acid ester. Preferred exipients are complexing agents, such as disodium-EDTA. The amount and type of excipient added is in accordance with the particular requirements and is generally in the range of from approximately 0.0001 to approximately 90% by weight.

A cyclodextrin as is referred to within the present invention is either an α-, β- or γ-cyclodextrin itself, a derivative thereof, e.g. a partially etherified derivative as e.g. a hydroxyalkyl ether or a mixture thereof. A random cyclodextrin does not automatically form an inclusion complex with any random pharmaceutical compound. Therefore the present invention relates preferably to such a cyclodextrin that meets the cavity needs of the pharmaceutical compound used. This may typically be accomplished by using either an α-, β- or γ-cyclodextrin itself or an appropriately substituted, α-, β- or γ- cyclodextrin or a mixture of the aforementioned cyclodextrins.

In another aspect of the present invention, a pharmaceutical composition comprising an ascomycin may additionally contain a second drug, preferably selected from an antiinfective drug and an antiinflammatoriy drug. Such a combination of drugs may be used to prevent complications and/or to improve the efficacy of said ascomycin used to treat said blepharitis. A preferred combination comprises an ascomycin and an antiinfective drug. Another preferred combination drug in the treatment of blepharitis may comprise an ascomycin and an antiinflammatory drug.

Accordingly, the invention may also relate to a method to treat blepharitis in a patient in need thereof, which method comprises the topical administration of a pharmaceutical composition comprising a therapeutic effective amount of an ascomycin, and a therapeutic effective amount of an antiinfective drug sufficient to prevent and/or treat an infection in said patient suffering from said blepharitis.

It further relates to a method to treat blepharitis in a patient in need thereof, which method comprises the topical administration of a pharmaceutical composition comprising a therapeutic effective amount of an ascomycin, and a therapeutic effective amount of an antiinflammatory drug sufficient to prevent and/or treat an inflammation in said patient suffering from said blepharitis.

In another preferred aspect of the present invention the treatment of blepharitis may be effected by the topical administration of a pharmaceutical composition comprising an ascomycin as the only pharmaceutically effective drug, also known as mono drug therapy.

Antiinflammatory drugs are typically selected from steroids, such as dexamethasone, fluorometholone , hydrocortisone, prednisolone; so-called non-steroidal anti-inflammatory drugs (NSAID) such as COX-inhibitors, e.g. diclofenac, ketorolac, and indomethacin. Preferred are NSAID, more preferred is diclofenac.

Antiinfective drugs are typically selected from from chloramphenicol, chlortetracycline, ciprofloxacin, gentamycin, neomycin, ofloxacin, polymyxin B and tobramycin. Preferred is ciprofloxacin and tobramycin, more preferred is tobramycin.

## Claims

1. Use of an ascomycin in the preparation of a pharmaceutical composition for the treatment of blepharitis.

2. Use of claim 1, wherein said ascomycin is selected from FK506; 33-epi-chloro-33-desoxy-ascomycin; {[1E-(1 R,3R,4R)]1 R,4S,5R,6S,9R,10E,13S,15S,16R,17S,19S,20S}-9-ethyl-6,16, 20-trihydroxy-4-[2-(4-hydroxy-3-methoxy-cyclohexyl)-1-methylvinyl]-15,17-dimethoxy-5,11,13,19-tetramethyl-3-oxa-22-aza-tricyclo[18.6.1.0(1,22)]heptacos-10-ene-2,8,21,27-tetraone; {1R,5Z,9S,12S-[1E-(1R,3R,4R)],13R,14S,17R,18E,21S,23S,24R,25S,27R}17-ethyl-1,14-dihydroxy-12-[2-(4-hydroxy-3-methoxy-cyclohexyl)-1-methylvinyl]-23,25-dimethoxy-13,19,21,27-tetramethyl-11,28-dioxa-4-aza-tricyclo[22.3.1.0(4,9)] octacosa-5,18-diene-2,3,10,16-tetraone, also known as 5,6-dehydro-ascomycin; imidazolylmethyloxyascomycin; 32-O-(1-hydroxyethylindol-5-yl)ascomycin, also known as Indolyl-ASC or L-732 531; and (32-deoxy-32-epi-N1-tetrazolyl)ascomycin, also known as ABT-281.

3. Use of claim 1, wherein said ascomycin is selected from FK506; and 33-epi-chloro-33-desoxy-ascomycin.

4. Use of claim 1, wherein said ascomycin is 33-epi-chloro-33-desoxy-ascomycin.

5. Use of claim 1, wherein said pharmaceutical composition is an ointment, cream, gel, or lotion, preferably an ointment.

6. Use according to claim 1-5, wherein said pharmaceutical composition is for topical administration.

7. Use of claim 6, wherein said administration is topically to the eyelid of a patient.

8. Method of treating blepharitis, which method comprises the administration of a pharmaceutical composition comprising a therapeutically effective amount of an ascomycin to the affected area of the patient suffering from blepharitis, said ascomycin being preferably administered to the eyelid of said patient.

9. Method of claim 8, wherein said blepharitis is staphylococcal blepharitis, seborrhoeic blepharitis or allergic blepharitis.

10. Method of claim 8, wherein said ascomycin is selected from any one of claims 2-4.

11. A pharmaceutical composition comprising an ascomycin for use in the treatment of blepharitis.

12. A topical pharmaceutical composition comprising an ascomycin, wherein said topical composition is selected from an ointment, cream, gel, and lotion, and wherein said ascomycin is for use in blepharitis.
